# EUROPEAN PATENT SPECIFICATION

(11) **EP 2 747 718 B1**
(45) Date of publication and mention of the grant of the patent: **01.07.2015**
(21) Application number: 11776838.2
(22) Date of filing: 26.08.2011
(51) Int. Cl.: A61F 5/02, A61F 5/37

(54) **DEVICE FOR IMPROVING THE POSTURE OF A USER**
VORRICHTUNG ZUR VERBESSERUNG DER HALTUNG EINES BENUTZERS
DISPOSITIF D'AMÉLIORATION DE LA POSTURE D'UN UTILISATEUR

(43) Date of publication of application: 02.07.2014
(73) Proprietor: Naef, Neda, 8910 Affoltern am Albis (CH)
(72) Inventor: Naef, Neda, 8910 Affoltern am Albis (CH)
(74) Representative: Osha Liang
(86) International application number: PCT/IB2011/001958
(87) International publication number: WO 2013/030610

(56) References cited:
- WO-A2-2005/086840
- GB-A- 2 159 058
- US-A1- 2004 118 411
- US-B1- 6 440 094

## Description

### BACKGROUND

### Field of the invention

The present invention relates to a device for improving the posture of a user, to a garment comprising such a device, as well as to a method for improving the posture of a user.

### Background Art

Persons may suffer from back problems due to a poor posture. In a poor posture, shoulders generally tend to be inclined forwards and the spine is not in proper alignment, with the consequence that the back muscles, ligaments and spinal joints are under extra stress. Back muscle strain can cause back pain.

On the other side, it is known that maintaining a proper posture, both in a static position such as sitting and during dynamic activities such as walking, allows to train muscles of the waist, of the abdomen and of the whole back.

A number of devices having the aim of improving the posture of a user are known. So, for example, document DE 19523672 A1 describes a device comprising two shoulder caps, each with two holding slits on the front and rear side respectively. The slits on the front side retain an elastic band, whilst those on the rear side are joined to a holding brace. However, the actions exerted by the elastic band and by the holding brace tend to cancel each other out. Furthermore, the shoulder caps are uncomfortable to wear.

Document WO2005/086840 A2 describes a device for improving the posture of a patient comprising a garment enveloping a portion of the torso and at least a portion of the upper arms. The garment is in the form of a T-shirt enveloping at least a portion of the patient's torso and at least a portion of the patient's upper arms. The garment includes in particular a pair of elongate, extendable tension straps releasably secured to a back side, to a waist portion and to shoulder portions of the garment. The tension straps are diagonally disposed relative to one another and are releasably secured to the back side of the garment. However, the garment is necessarily stretched by the action of the tension straps incorporated within and cannot be maintained in position and worn in a comfortable manner.

Document US 6 387 067 B1 describes a shoulder support device for facilitating the correct posture of the shoulders, the device comprising an elongate, resilient base member fitting about the circumference of a central or upper chest portion of a wearer, and a pair of spaced apart shoulder strap members secured to the base member. However, the shoulder straps act on an area between the shoulders and the neck which does not permit to exert an effective biasing action of the shoulders in the rearward direction, as needed to ensure a proper posture without making the device too structurally complicated or too heavy to wear.

Document US 2004/118411 A1 describes a form-correcting chest belt, forcing both arms, shoulders and chests to move in co-relation with one another, which is used together with a form-correcting waist belt, forcing the vertebral column to become stiff and the upper and lower parts of the body to move in co-relation with each other, and together with form-correcting shoes or foot angle supporter, which semi-force body weight center to be located in the middle of the foot length, thereby helping players play easily in the right forms the sports using rackets or bats or clubs, such as tennis, badminton, baseball and golf.

One objective of the present invention is that of providing a device for improving the posture which is effective, structurally simple and comfortable to the user.

### SUMMARY OF THE INVENTION

The Applicant has surprisingly found that the posture of a user may be improved by exerting a pressure on the outermost portion of the user's shoulders and the uppermost part of the user's arms (*i.e.* on the muscles surrounding the humeral head) in a simple manner.

In one aspect thereof, the present invention comprises a device for improving the posture of a user, comprising:
a first armband and a second armband intended to be worn at the uppermost portion (proximal portion) of a left arm and a right arm, respectively;
at least one first strip fixed to a front part of the first armband and to a back part of the second armband; and
at least one second strip fixed to a front part of the second armband and to a back part of the first armband.

In the present description and in the following claims, the terms "front" and "back" refer to the position of an element of the device with reference to the position assumed by the element when the device is worn by the user.

When the posture is not correct, the device of the invention advantageously exerts a pressure on the outer end of each shoulder and on the uppermost part of each arm (*i.e.* on the proximal part of each arm), thus inducing a proper posture in an effective manner. By acting on the outer end of each shoulder and on the uppermost part of each arm (i.e. on the muscles surrounding the humeral head), in fact, the shoulders are biased in a rearward and downward direction by a limited force when compared to the force required to obtain a comparable effect in the prior art devices acting on innermost parts of the shoulders, generally in an intermediate position between the shoulder and the neck. In particular, when a user puts on the device of the invention, the at least one first strip and the at least one second strip, fixed on the first armband and the second armband, pull the user's shoulders rearwards and downwards.

Advantageously, the device exerts its action of biasing the user's shoulders in a rearward and downward direction without altering the free motion of all joints of the back, neck, shoulders and arms of the user.

Furthermore, the device of the invention is structurally simple.

Furthermore, since the at least one first strip and the at least one second strip of the device are fixed to the first and second armbands, which in turn, when in use, are worn at the uppermost portion of respective arms, the at least one first strip and the at least one second strip are kept firmly in position and do not slip from the shoulders.

Furthermore, the device of the invention is comfortable to wear and can be easily worn and used either as such or incorporated in a garment.

On the front part of the user's body, the at least one first strip and the at least one second strip, each extending from the front part of a respective armband, partially cover only the user's shoulders and back, while leaving the user's waist free.

On the back part of the user's body, the at least one first strip and the at least one second strip may cross to each other. However, when the device comprises, as described more in detail in the following, two first strips and two second strips, the two first strips may be connected together either directly or by a connecting member and the two second strips may be connected together either directly or by the same connecting member.

Each of the first and second armbands may be sized and shaped to fit around the uppermost portion of the arm.

According to one embodiment, each of the first armband and second armband is made of elastic material, such as for example a natural or synthetic elastic material or mixtures thereof.

According to one embodiment, each of the first armband and of the second armband comprises a continuous band of textile material, for example an elastic material.

The textile material may be for example covered by a layer of silicone or other suitable elastic material.

According to an alternative embodiment, each of said first armband and second armband comprises a plurality of ribbons, *i.e.* a plurality of narrow strips arranged substantially parallel to each other. The ribbons may be made of elastic material.

According to one embodiment, each of the first armband and second armband comprises a tailored length of material, either in the form of a continuous band of material or in the form of a plurality of ribbons, fixed together at the free ends thereof, for example by sewing. However, according to another embodiment, the free ends of the length of material used to prepare the first and second armbands are fixed together by releasable fixing means, such as for example strap members, Velcro fasteners, zippers, buttons or other means suitable for the purpose. The releasable fixing means advantageously both allow an adjustment of the size of the first and second armbands and make easier the disengagement of the first and second armbands when the user wishes to take the device off.

According to one embodiment, each of said first armband and second armband comprises a plurality of ribbons, for example at least two ribbons, or at least three ribbons.

According to one embodiment, each of the first armband and second armband has a width comprised between 1 cm and 8 cm.

The width of each of the first and second armbands may be constant.

According to an alternative embodiment, the width of the first armband and second armband may be minimum at the internal part of the user's arm and may progressively increase towards the external part of the user's arm. In this way, the comfort is advantageously further improved without impairing the resistance of the device.

According to one embodiment, each of the at least one first strip and at least one second strip is sized and shaped to fit on the shoulders and on the back of the user with a predetermined tension.

According to one embodiment, each of said at least one first strip and at least one second strip comprises a continuous band of textile material, for example an elastic material, such as for example a natural or synthetic elastic material or mixtures thereof.

According to a further embodiment, each of said first and second armbands and/or each of said first and second strips may comprise composite materials or combinations of different materials. The composite material used for the armbands may for example comprise a soft material at the part intended to be in contact with the internal part of the user's arm.

According to one embodiment, the at least one first strip comprises a plurality of first ribbons, *i.e.* a plurality of narrow strips arranged substantially parallel to each other. Similarly, the at least one second strip may comprise a plurality of second ribbons. The first and second ribbons may be of elastic material.

According to one embodiment, each of said plurality of first ribbons and plurality of second ribbons comprises at least two ribbons, for example at least three ribbons.

Each one of the at least one first strip and at least one second strip may have for example a width comprised between 2 cm and 7 cm.

The width of each of the at least one first strip and at least one second strip may be constant. According to a further embodiment, the width of the at least one first strip and at least one second strip is maximum at the fixing points with the first and second armbands, and for example of at least 4 cm. However, alternative designs of the at least one first strip and at least one second strip and of the width thereof are also possible.

The at least one first strip and the at least one second strip may be fixed to the first armband and to the second armband by sewing or by any other suitable fixing means. The fixing means may be releasable, which advantageously makes easier their disengagement when the user wishes to take the device off. The fixing means may comprise strap members, Velcro fasteners and other fasteners suitable for the purpose. When the device comprises releasable fixing means, the first strip and second strip may be for example made of a non-elastic material or of a slightly elastic material, such as for example cotton.

According to one embodiment, both the strips and the armbands are made of the same material. However, different combinations are possible. So, for example, first and second strips each comprising a plurality of ribbons may be combined with first and second armbands made of a single continuous band of textile material, or vice versa.

According to one embodiment, the device for improving the posture may comprise two first strips and two second strips. The two first strips may be connected together at one free end thereof by sewing. Similarly, the two second strips may be connected together at one free end thereof by sewing. However, as an alternative embodiment, the device may further comprise a first connecting member connecting the two first strips together and the two second strips together. In this way, the forces pulling the user's shoulders may be still better distributed. Furthermore, in this way the first strips and the second strips are advantageously better kept in position in the back part of the device. Said first connecting member and the two first strips and two second strips may be made of the same material. For example, the first connecting member, the two first strips and the two second strips are made in a single piece of a textile material. Alternatively, the first connecting member may be made of a different material than the two first strips and two second strips to better adjust the forces pulling the user's shoulders.

According to one embodiment, the device further comprises a third strip fixed, at the free end thereof, to the back part of the first armband and to the back part of the second armband. The third strip may be also fixed, at an intermediate portion thereof, to the at least one first strip and to the at least one second strip in.

In this way, the at least one first strip and the at least one second strip are advantageously maintained in position in a still more stable manner.

When the device comprises two first strips and two second strips connected together by a first connecting member as described above, the third strip may be connected to said first connecting member.

The device may comprise a second connecting member connecting the at least one first strip and the at least one second strip together. When the at least one first strip and the at least one second strip cross each other, the second connecting member can hold together the at least one first strip and the at least one second strip at the crossing point thereof.

According to one embodiment, the device further comprises a belt and at least one fourth strip fixed to a back part of the belt and to said second connecting member or, when the latter is not present, directly to either of the first and second strips or to both first and second strips.

However, a belt and at least a fourth strip may be envisaged also in combination with the embodiment according to which a first connecting member connects two first strips together and two second strips together. In particular, when the two first strips and two second strips are connected together by a first connecting member, the at least fourth strip fixed to the back part of the belt can be connected to said first connecting member.

When a belt and at least a fourth strip are present, the device is advantageously still better kept in position in the back portion of the device.

According to one embodiment, the device comprises at least one pair of adjusting members intended to adjust the length of the at least one first strip and of the at least one second strip and/or the size of the first armband and of the second armband.

According to one embodiment, the device comprises a first pair of adjusting members arranged on the first armband and on the second armband and/or a second pair of adjusting member arranged on the at least one first strip and on the at least one second strip.

In this way, it is advantageously possible for the user to adjust and thus tailor the size and/or length of all elements making part of the device, thus adjusting the action exerted by the device on the shoulders and/or on the arms according to his/her needs.

According to one embodiment, the adjusting members of the first pair of adjusting members are arranged in the region of the respective armbands intended to cover the external part of the arm.

The adjusting members may comprise strap members, Velcro fasteners, zippers, buttons or other means suitable for the purpose or any other means suitable for the purpose.

The device may be covered by a lining fabric, which may further enhance the user's comfort.

The device may be used as such in order to improve the user's posture. However, the device is suitable to be incorporated in a garment.

According to a further aspect thereof, the present invention provides a garment comprising the device according to one or more of the embodiments defined above.

The garment may be a shirt, a T-shirt, a pullover or any other type of garment.

By way of illustrative example, the garment may be made of cotton or of cotton and elastic fibers.

The device may be incorporated in the garment by sewing, by glue or by means of any kind of fixing means or fasteners suitable for the purpose. The garment may comprise a plurality of fixing means or fasteners arranged at different portions of the device and intended to attach the device to garment. The garment may comprise two layers of fabric or a lining within which the device may be incorporated. However, the device can be also incorporated inside or outside or partially inside and partially outside the garment.

The device may be detachable from the garment. In this case, releasable fixing means may be provided to fix the device to the garment. Embodiments of releasable fixing means comprise for example strap members and Velcro fasteners.

According to a further aspect thereof, the present invention provides a method to improve the posture of a user. The method comprises levering the user's shoulders rearwards and downwards by: arranging a first armband and a second armband around the uppermost portion (i.e. proximal portion) of the user's arms; crossing to each other on the user's back at least one first strip and at least one second strip; fixing the at least one first strip to a front part of the first armband and to a back part of the second armband; and fixing the at least one second strip to a back part of the first armband and to a front part of the second armband.

When only one first strip is envisaged, fixing the first strip may comprise fixing the first strip to a back part of the second armband directly.

When the at least one first strip comprises two first strips, these may be fixed to the first and second armbands by interposition of a first connecting member connecting the two first strips together. The at least one second strip, when it comprises two second strips, may be fixed to the first and second armbands in the same manner.

Furthermore, when the at least one first strip comprises two first strips and the at least one second strip comprises two second strips, crossing to each other on the user's back the at least one first strip and at least one second strip may comprise crossing an assembly comprising the two first strips and an assembly comprising the two second strips on the user's back, optionally by interposition of a connecting member connecting all strips at a common crossing point. According to embodiments of the method of the invention, the at least one first strip and the at least one second strip, as well as the first armband and the second armband and further optional features may be like the optional features disclosed with reference to any of the above-mentioned embodiments of the device.

Other aspects and advantages of the disclosure will be apparent from the following description and the appended claims.

### BRIEF DESCRIPTION OF DRAWINGS

Figure 1 and Figure 2 illustrate a front view and, respectively, a back view of a device for improving the posture of a user according to an embodiment of the present invention.
Figure 3 illustrates a back view of a device for improving the posture of a user according to an embodiment of the present invention.
Figure 4 illustrates a back view of a device for improving the posture of a user according to an embodiment of the present invention.
Figure 5 and Figure 6 illustrate a front view and, respectively, a back view of a device for improving the posture of a user according to an embodiment of the present invention.
Figure 7 and Figure 8 illustrate a front view and, respectively, a back view of a device for improving the posture of a user according to an embodiment of the present invention.

### DETAILED DESCRIPTION OF PREFERRED EMBODIMENTS

Embodiments of the present invention relating to a device for improving the posture of a user will now be described in detail with reference to the accompanying Figures. Like elements in the various Figures may be denoted by like reference numbers.

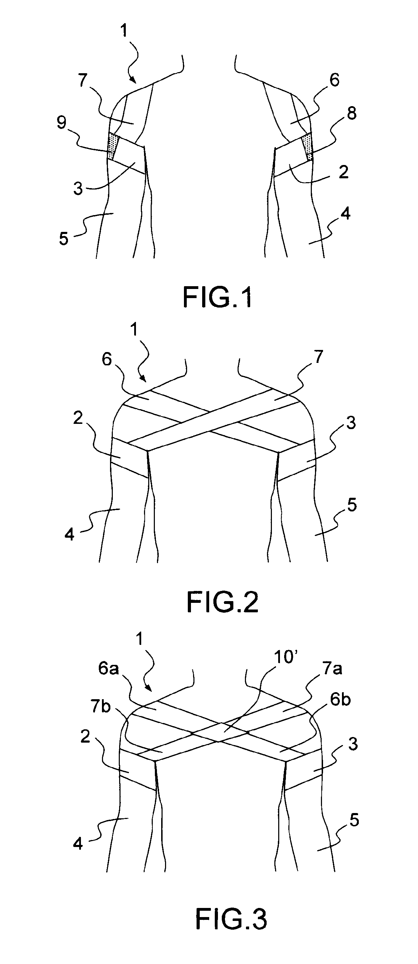

An embodiment of the device is shown in Figure 1 and 2. In these Figures, the device is indicated by reference number 1. The device 1 comprises a first armband 2 and a second armband 3, each comprising a continuous band of textile material, for example comprising elastic synthetic fibers.

The first armband 2 is intended to be worn at the uppermost portion of the left arm 4, while the second armband 3 is intended to be worn at the uppermost portion of the right arm 5. To this purpose, each of the first armband 2 and second armband 3 is sized and shaped to fit around the uppermost portion of the arm.

In the embodiment shown in Figures 1 and 2, the width of each armband 2, 3 is of about 6 cm. However, according to further embodiments, the width of each band may be minimum at the internal part of the user's arm, for example of about 2 cm, and may progressively increase towards the external part of the arm, for example up to about 6 cm so as to advantageously increase the comfort while ensuring a good resistance of the device.

Furthermore, the device 1 comprises a first strip 6 fixed to a front part of the first armband 2 and to a back part of the second armband 3 and a second strip 7 fixed to a front part of the second armband 3 and to a back part of the first armband 2.

In this embodiment, the first strip 6 and the second strip 7 are continuous bands of elastic material, for example comprising elastic synthetic fibers, for example having a width of about 5 cm, and are fixed by sewing to the first armband 2 and to the second armband 3.

As shown in Figures 1 and 2, the first strip 6 and the second strip 7 partially cover only the shoulders and the upper portion of the back of the user and leave the waist free, while the first strip 6 and second strip 7 cross to each other on the back part of the user's body.

Each of the first strip 6 and second strip 7 is sized and shaped to fit on the shoulders and on the back of the user with a predetermined tension.

Adjusting members 8 and 9, for example in the form of Velcro fasteners, are provided in the first armband 2 and, respectively, in the second armband 3, in each armband in the region intended to cover the external part of the arm. The adjusting members 8 and 9 are intended to adjust the size of each armband 2, 3 according to the user's needs.

In Figure 3 a further embodiment of the device 1 is shown. According to this embodiment, the device 1 comprises two first strips 6a, 6b and two second strips 7a, 7b. Furthermore, in this embodiment the device 1 further comprises a connecting member 10', for example of elastic synthetic fibers, connecting the two first strips 6a, 6b together and the two second strips 7a, 7b together.

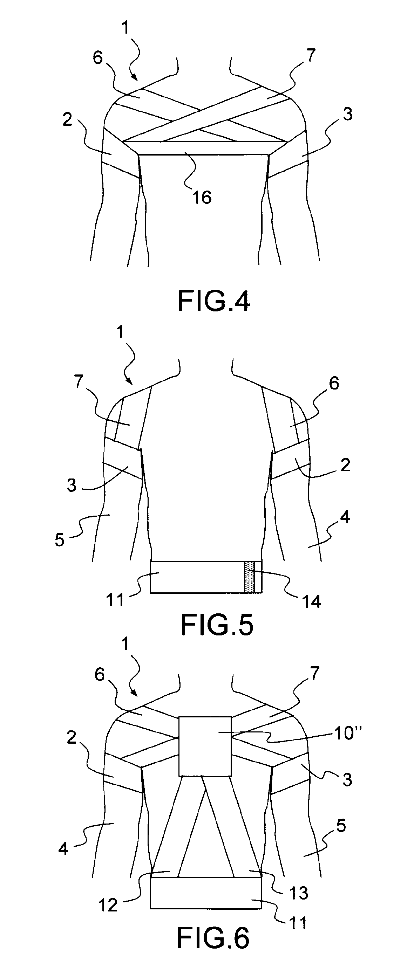

In Figure 4 a further embodiment of the device 1 is shown. In this embodiment, the device 1 further comprises, in addition to the elements already described with reference to the embodiment of Figures 1 and 2, a third strip 16. The third strip 16 is fixed for example by sewing, at the free ends thereof, to the back part of the first armband 2 and to the back part of the second armband 3. However, according to further embodiments, the such a third strip may be fixed, at an intermediate portion thereof, also to the first strip 6 and to the second strip 7.

In Figures 5 and 6 a further embodiment of the device 1 is shown. The device 1 shown in these figures further comprises a belt 11 comprising a continuous band, for example of elastic synthetic fibers.

The belt 11 comprises a tailored length of material sized and shaped to fit around the user's body and fixed together at the free ends thereof by adjusting means 14, such as for example a Velcro fastener.

Thanks to the adjusting means 14, the belt 11 can be easily disengaged when the user intends to take the device off.

Furthermore, the device 1 comprises a fourth strip 12 and a fifth strip 13 fixed to a connecting member 10" and to a back portion of the belt 11.

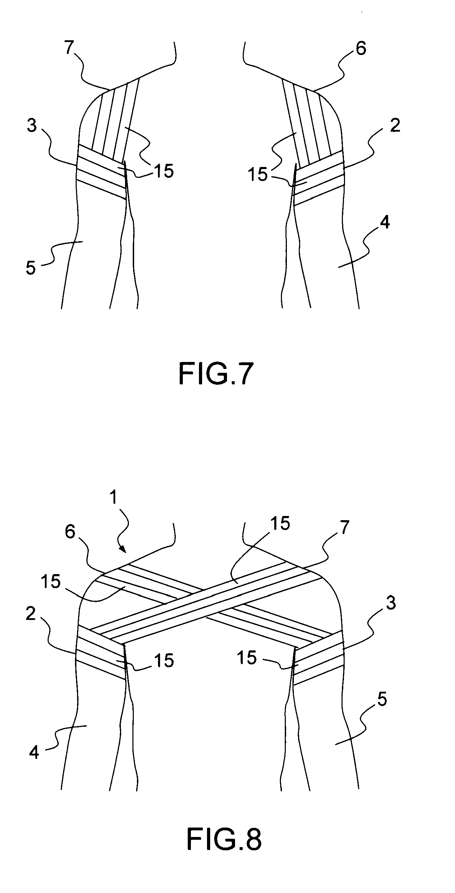

For example, the connecting member 10" is a keeper, enabling to hold the first strip 6 and the second strip 7, together with the fourth strip 12 and the fifth strip 13.In the embodiment shown in Figures 7 and 8, each of the first armband 2 and the second armband 3 comprises a plurality of ribbons 15, for example made of elastic synthetic fibers. In a similar manner, each of the first strip 6 and second strip 7 comprises a plurality of ribbons 15, for example made of elastic synthetic fibers, which are fixed to the first armband 2 and second armband 3, for example by sewing.

In each of the above-mentioned embodiments of the device 1, the user can easily put on the device 1 by introducing the left arm in the armband 2 up to the uppermost portion of the arm, the right arm 5 in the second armband 3 up to the uppermost portion of the arm and then by sliding the first strip 6 and the second strip 7 onto the shoulders. In this way, the user's shoulders are pushed rearwards and downwards by the action exerted by the first strip 6 and the second strip 7, which are in turn pulled and kept in position by the user's arms through the second armband 3 and the first armband 2, respectively.

While the invention has been described with respect to a limited number of embodiments, those skilled in the art, having benefit of this disclosure, will appreciate that other embodiments can be devised, such as for example embodiments based on alternative materials or on alternative ways to fix the strips to the armbands.

## Claims

1. A device (1) for improving the posture of a user, comprising:
- a first armband (2) and a second armband (3) intended to be worn at the uppermost portion of a left arm (4) and a right arm (5), respectively;
**characterised by**
- at least one first strip (6) fixed to a front part of the first armband (2) and to a back part of the second armband (3); and
- at least one second strip (7) fixed to a front part of the second armband (3) and to a back part of the first armband (2).

2. The device (1) according to claim 1, wherein each of said first armband (2) and second armband (3) comprises a continuous band of textile material.

3. The device (1) according to claim 1 or 2, wherein each of said at least one first strip (6) and at least one second strip (7) comprises a continuous band of textile material.

4. The device (1) according to any one of the preceding claims, wherein each of said first armband (2) and second armband (3) comprises a plurality of ribbons (15).

5. The device (1) according to any one of the preceding claims, wherein each of said first armband (2) and second armband (3) and each of said at least one first strip (6) and at least one second strip (7) is made of an elastic material.

6. The device (1) according to any one of the preceding claims, wherein the at least one first strip (6) comprises a plurality of ribbons (15) fixed to a front part of the first armband (2) and to a back part of the second armband (3), and the at least one second strip (7) comprises a plurality of ribbons (15) fixed to a front part of the second armband (3) and to a back part of the first armband (2).

7. The device (1) according to any one of the preceding claims, further comprising a third strip (16) fixed to the back part of the first armband (2) and to the back part of the second armband (3).

8. The device (1) according to any one of the preceding claims, wherein the device (1) comprises:
- two first strips (6a, 6b) and two second strips (7a, 7b); and
- a first connecting member (10') connecting the two first strips (6a, 6b) together and the two second strips (7a, 7b) together.

9. The device (1) according to claim 8, further comprising:
- a belt (11); and
- at least one fourth strip (12, 13) fixed to said first connecting member (10') and to a back part of the belt (11).

10. The device (1) according to claim 8, wherein the two first strips (6a, 6b), the two second strips (7a, 7b) and the first connecting member (10') are made in a single piece of a same material.

11. The device (1) according to any one of the preceding claims, further comprising at least one pair of adjusting members intended to adjust the length of the at least one first strip (6) and the at least one second strip (7).

12. The device (1) according to any one of the preceding claims, further comprising at least one pair of adjusting members (8, 9) intended to adjust the size of the first armband (2) and the second armband (3).

13. A garment comprising a device (1) according to any one of the preceding claims.

14. The garment of claim 13, wherein the device (1) is detachable from the garment.

15. A method for improving the posture of a user, comprising levering the user's shoulders rearwards and downwards by:
- arranging a first armband (2) and a second armband (3) around the uppermost portion of the user's arms;
- crossing to each other on the user's back at least one first strip (6) and at least one second strip (7);
- fixing the at least one first strip (6) to a front part of the first armband (2) and to a back part of the second armband (3); and
- fixing the at least one second strip (7) to a back part of the first armband (2) and to a front part of the second armband (3).

## Patentansprüche

1. Vorrichtung (1) zum Verbessern der Körperhaltung eines Nutzers, wobei die Vorrichtung umfasst:
- eine erste Armbinde (2) und eine zweite Armbinde (3), die dafür bestimmt sind, an dem obersten Abschnitt eines linken Arms (4) bzw. eines rechten Arms (5) getragen zu werden;
**dadurch gekennzeichnet, dass**
- mindestens ein erster Streifen (6) an einem vorderen Teil der ersten Armbinde (2) und an einem hinteren Teil der zweiten Armbinde (3) befestigt ist; und
- mindestens ein zweiter Streifen (7) an einem vorderen Teil der zweiten Armbinde (3) und an einem hinteren Teil der ersten Armbinde (2) befestigt ist.

2. Vorrichtung (1) nach Anspruch 1, wobei sowohl die erste Armbinde (2) als auch die zweite Armbinde (3) ein ununterbrochenes Band aus Textilmaterial umfasst.

3. Vorrichtung (1) nach Anspruch 1 oder 2, wobei jeder des mindestens einen ersten Streifens (6) und des mindestens einen zweiten Streifens (7) ein durchgängiges Band aus Textilmaterial umfasst.

4. Vorrichtung (1) nach einem der vorhergehenden Ansprüche, wobei jede der ersten Armbinde (2) und der zweiten Armbinde (3) mehrere Stränge (15) umfasst.

5. Vorrichtung (1) nach einem der vorhergehenden Ansprüche, wobei jede der ersten Armbinde (2) und der zweiten Armbinde (3) und jeder des mindestens einen ersten Streifens (6) und des mindestens einen zweiten Streifens (7) aus einem elastischen Material hergestellt ist.

6. Vorrichtung (1) nach einem der vorhergehenden Ansprüche, wobei der mindestens eine erste Streifen (6) mehrere Stränge (15) umfasst, die an einem vorderen Teil der ersten Armbinde (2) und an einem hinteren Teil der zweiten Armbinde (3) befestigt sind, und wobei der mindestens eine zweite Streifen (7) mehrere Stränge (15) umfasst, die an einem vorderen Teil der zweiten Armbinde (3) und an einem hinteren Teil der ersten Armbinde (2) befestigt sind.

7. Vorrichtung (1) nach einem der vorhergehenden Ansprüche, die ferner einen dritten Streifen (16) umfasst, der an dem hinteren Teil der ersten Armbinde (2) und an dem hinteren Teil der zweiten Armbinde (3) befestigt ist.

8. Vorrichtung (1) nach einem der vorhergehenden Ansprüche, wobei die Vorrichtung (1) umfasst:
- zwei erste Streifen (6a, 6b) und zwei zweite Streifen (7a, 7b); und
- ein erstes Verbindungselement (10'), das die zwei ersten Streifen (6a, 6b) miteinander und die zwei zweiten Streifen (7a, 7b) miteinander verbindet.

9. Vorrichtung (1) nach Anspruch 8, die ferner umfasst:
- einen Gürtel (11); und
- mindestens einen vierten Streifen (12, 13), der an dem ersten Verbindungselement (10') und an einem hinteren Teil des Gürtels (11) befestigt ist.

10. Vorrichtung (1) nach Anspruch 8, wobei die zwei ersten Streifen (6a, 6b), die zwei zweiten Streifen (7a, 7b) und das erste Verbindungselement (10') in einem einzelnen Teil aus einem selben Material hergestellt sind.

11. Vorrichtung (1) nach einem der vorhergehenden Ansprüche, die ferner mindestens ein Paar Einstellelemente umfasst, die dafür bestimmt sind, die Länge des mindestens einen ersten Streifens (6) und des mindestens einen zweiten Streifens (7) einzustellen.

12. Vorrichtung (1) nach einem der vorhergehenden Ansprüche, die ferner mindestens ein Paar Einstellelemente (8, 9) umfasst, die dafür bestimmt sind, die Größe der ersten Armbinde (2) und der zweiten Armbinde (3) einzustellen.

13. Kleidungsstück, das eine Vorrichtung (1) nach einem der vorhergehenden Ansprüche umfasst.

14. Kleidungsstück nach Anspruch 13, wobei die Vorrichtung (1) von dem Kleidungsstück gelöst werden kann.

15. Verfahren zum Verbessern der Körperhaltung eines Nutzers, wobei das Verfahren umfasst, dass eine Hebelwirkung auf die Schultern des Nutzers nach hinten und nach unten ausgeübt wird durch:
- Anordnen einer ersten Armbinde (2) und einer zweiten Armbinde (3) um den obersten Abschnitt der Arme des Nutzers;
- Kreuzen mindestens eines ersten Streifens (6) und mindestens eines zweiten Streifens (7) auf dem Rücken des Nutzers miteinander;
- Befestigen des mindestens einen ersten Streifens (6) an einem vorderen Teil der ersten Armbinde (2) und an einem hinteren Teil der zweiten Armbinde (3); und
- Befestigen des mindestens einen zweiten Streifens (7) an einem hinteren Teil der ersten Armbinde (2) und an einem vorderen Teil der zweiten Armbinde (3).

## Revendications

1. Dispositif (1) destiné à améliorer la posture d'un utilisateur, comprenant :
- un premier brassard (2) et un deuxième brassard (3) destinés à être portés sur la partie la plus haute d'un bras gauche (4) et d'un bras droit (5), respectivement ;
**caractérisé par**
- au moins une première bande (6) fixée à une partie avant du premier brassard (2) et à une partie arrière du deuxième brassard (3) ; et
- au moins une deuxième bande (7) fixée à une partie avant du deuxième brassard (3) et à une partie arrière du premier brassard (2).

2. Dispositif (1) selon la revendication 1, dans lequel chacun desdits premier brassard (2) et deuxième brassard (3) comprend une bande continue de matériau textile.

3. Dispositif (1) selon la revendication 1 ou la revendication 2, dans lequel chacune desdites au moins une première bande (6) et au moins une deuxième bande (7) comprend une bande continue de matériau textile.

4. Dispositif (1) selon l'une quelconque des revendications précédentes, dans lequel chacun desdits premier brassard (2) et deuxième brassard (3) comprend une pluralité de rubans (15).

5. Dispositif (1) selon l'une quelconque des revendications précédentes, dans lequel chacun desdits premier brassard (2) et deuxième brassard (3) et chacune desdites au moins une première bande (6) et au moins une deuxième bande (7) est fabriqué d'un matériau élastique.

6. Dispositif (1) selon l'une quelconque des revendications précédentes, dans lequel l'au moins une première bande (6) comprend une pluralité de rubans (15) fixés à une partie avant du premier brassard (2) et à une partie arrière du deuxième brassard (3), et l'au moins une deuxième bande (7) comprend une pluralité de rubans (15) fixés à une partie avant du deuxième brassard (3) et à une partie arrière du premier brassard (2).

7. Dispositif (1) selon l'une quelconque des revendications précédentes, comprenant en outre une troisième bande (16) fixée à la partie arrière du premier brassard (2) et à la partie arrière du deuxième brassard (3).

8. Dispositif (1) selon l'une quelconque des revendications précédentes, dans lequel le dispositif (1) comprend :
- deux premières bandes (6a, 6b) et deux deuxièmes bandes (7a, 7b) ; et
- un premier élément de liaison (10') reliant les deux premières bandes (6a, 6b) ensemble et les deux deuxièmes bandes (7a, 7b) ensemble.

9. Dispositif (1) selon la revendication 8, comprenant en outre :
- une ceinture (11) ; et
- au moins une quatrième bande (12, 13) fixée audit premier élément de liaison (10') et à une partie arrière de la ceinture (11).

10. Dispositif (1) selon la revendication 8, dans lequel les deux premières bandes (6a, 6b), les deux deuxièmes bandes (7a, 7b) et le premier élément de liaison (10') sont fabriqués d'une seule pièce d'un même matériau.

11. Dispositif (1) selon l'une quelconque des revendications précédentes, comprenant en outre au moins une paire d'éléments d'ajustage destinés à ajuster la longueur de l'au moins une première bande (6) et de l'au moins une deuxième bande (7).

12. Dispositif (1) selon l'une quelconque des revendications précédentes, comprenant en outre au moins une paire d'éléments d'ajustage (8, 9) destinés à ajuster la taille du premier brassard (2) et du deuxième brassard (3).

13. Vêtement comprenant un dispositif (1) selon l'une quelconque des revendications précédentes.

14. Vêtement selon la revendication 13, dans lequel le dispositif (1) est séparable du vêtement.

15. Procédé pour améliorer la posture d'un utilisateur, comprenant la manipulation des épaules de l'utilisateur vers l'arrière et vers le bas par :
- l'agencement d'un premier brassard (2) et d'un deuxième brassard (3) autour de la partie la plus élevée des bras de l'utilisateur ;
- le croisement l'une avec l'autre d'au moins une première bande (6) et d'au moins une deuxième bande (7) sur le dos de l'utilisateur ;
- la fixation de l'au moins une première bande (6) à une partie avant du premier brassard (2) et à une partie arrière du deuxième brassard (3) ; et
- la fixation de l'au moins une deuxième bande (7) à une partie arrière du premier brassard (2) et à une partie avant du deuxième brassard (3).
